(19) Europäisches Patentamt
European Patent Office
Office européen des brevets

(11) Publication number: **0 239 801 B1**

(12) # EUROPEAN PATENT SPECIFICATION

(45) Date of publication of patent specification: **07.12.94** (51) Int. Cl.⁵: **C12N 15/29**

(21) Application number: **87102855.1**

(22) Date of filing: **27.02.87**

(54) **Plant cells resistant to herbicidal glutamine synthetase inhibitors.**

(30) Priority: **27.02.86 US 833156**

(43) Date of publication of application:
**07.10.87 Bulletin 87/41**

(45) Publication of the grant of the patent:
**07.12.94 Bulletin 94/49**

(84) Designated Contracting States:
**AT BE CH DE ES FR GB GR IT LI LU NL SE**

(56) References cited:
**EP-A- 0 037 723**
**WO-A-84/02913**
**WO-A-84/02920**
**WO-A-86/02097**

**JOURNAL OF MOLECULAR AND APPLIED GENETICS, vol. 2, 1984, pages 621-635, Raven-Press, New York, US; G. DONN et al.: "Herbicide-resistant alfalfa cells: Anexample of gene amplification in plants"**

(73) Proprietor: **THE GENERAL HOSPITAL CORPORATION**
**55 Fruit Street**
**Boston, MA 02114 (US)**

(72) Inventor: **Goodman, Howard M., Prof.**
**10 The Ledges Road**
**Newton Center Massachusetts 02159 (US)**
Inventor: **Donn, Günter, Dr.**
**Sachsenring 35**
**D-6238 Hofheim am Taunus (DE)**

(74) Representative: **Meyer-Dulheuer, Karl-Hermann, Dr. et al**
**Hoechst AG,**
**Werk Kalle-Albert,**
**Zentrale Patentabteilung KA**
**D-65174 Wiesbaden (DE)**

EP 0 239 801 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

BIOLOGICAL ABSTRACTS/RRM, abstract no. 29054399, Biological AbstractsInformation Service, Philadelphia, US; G.M. CORRUZI et al.: "Cloning of genesinvolved in higher plant nitrogen assimilation", & JOURNAL OF CELLULARBIOCHEMISTRY SUPLEMENT (US), 1985, vol. 0, no. 9, Part C, p. 233

CHEMICAL ABSTRACTS, vol. 105, 1986, abstract no. 128628q, Columbus, Ohio, US;E. TISCHER et al.: "Nucleotide sequence of an alfalfa glutamine synthetasegene", & MGG. MOL. GEN. GENET. 1986, 203(2), 221-9

THE EMBO JOURNAL, vol. 3, no. 1, 1984, pages 65-71; P.G. SANDERS et al.:"Amplification and cloning of the Chinese hamster glutamine synthetase gene"

IDEM

CHEMICAL & ENGINEERING NEWS, 22nd June 1981, pages 33-44; J.L. FOX: "Plant-molecular biology beginning to flourish"

GENE, vol. 41, no. 1, 1986, pages 47-57, Elsevier Science Publishers B.V.,Amsterdam, NL; P.P. CHEE et al.: "Expression of a bean storage protein'phaseolin minigene' in foreign plant tissues"

CHEMICAL ABSTRACTS, vol. 102, no. 17, 29th April 1985, pages 146-147, abstractno. 144097j, Columbus, Ohio, US; J.V. CULLIMORE et al.: "Glutamine synthetaseof Phaseolus vulgaris L.: organ-specific expression of a multigene family", &J. MOL. APPL. GENET. 1984, 2(6), 589-99

NATURE, vol. 293, no. 5830, 24th September 1981, pages 265-270, MacmillanJournals Ltd, Chesham, Bucks, GB; E.C. COCKING et al.: "Aspects of plantgenetic manipulation

BIOLOGICAL ABSTRACTS, vol. 79, 1985, abstract no. 86213, Biological AbstractsInformation Service, Philadelphia, US; G. RICCARDI et al.: "Cloning of theglutamine synthetase (EC6.3.1.2) gene from Spirulina platensis", & PLANT MOL.BIOL. 4(2/3), 1985, pages 133-136

CHEMICAL ABSTRACTS, vol. 103, 1985, page 213, abstract no. 100292k, Columbus,Ohio, US; S.M. RIDLEY et al.: "Effects of phosphinothricin on the isoenzymes ofglutamine synthetase isolated from plant species which exhibit varying degreesof susceptibility to the herbicide", & PLANT SCI. (LIMERICK, IREL.) 1985,39(1), 31-6

## Description

### TECHNICAL FIELD

The present invention relates to the use of recombinant DNA technology for the transformation of plant cells and, more specifically, the design and construction of plant cells which are resistant to herbicidal plant glutamine synthetase inhibitors, such as phosphinothricin.

### BACKGROUND ART

Glutamine Synthetase (GS) is a plant enzyme which has a central role in the assimilation of ammonia and in the regulation of nitrogen metabolism. Since in most plants glutamine synthetase is, via the glutamine synthetase/glutamate synthase pathway (Fig. 1), the only efficient way to detoxify ammonia released by nitrate reduction, amino acid degradation or photorespiration, plants are very susceptible to potent inhibitors of glutamine synthetase.

One of the most potent glutamine synthetase inhibitors known at present is phosphinothricin (1) (hereinafter PPT):

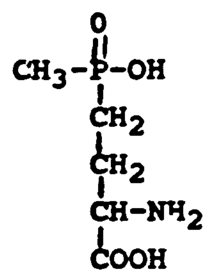

PPT is a glutamic acid analogue. The compound was initially isolated from a tripeptide antibiotic produced by Streptomyces viridochromogenes (Bayer, E. et al., Helvetica Chimica Acta, 55:224 (1972), see also German Patent DOS 2717440, Hoechst, A.G.). PPT is a potent competitive inhibitor of glutamine synthetase from E. coli with a $K_i$ of 0.0059 mM.

Schwerdtle, F. (Zeitschrift fur Pflanzen-Krankheiten and Pflanzenschutz, IX, 431-440 (1981)) demonstrated that PPT is a non-selective foliar herbicide for the control of undesirable mono- and dicotyledonous plants in orchards, vineyards, plantations with minimum tillage, direct drilling, and as a harvest aid. Field trials in West Germany, Spain, South Africa, U.S.A. and Japan showed that most dicotyledonous weeds were well controlled. For monocotyledonous weeds somewhat higher quantities were needed for good control. Leason, M. et al., (Phytochemistry, Volume 21:855-857 (1982)) demonstrated that PPT is a mixed competitive inhibitor of pea leaf glutamine synthetase with an apparent $K_i$ value of 0.073 mM.

It would be of great interest to be able to confer resistance to PPT, as well as to other GS inhibitors to selected plants, since herbicidal selectivity is quite crucial in any commercially useful herbicide PPT, as indicated, is non-selective.

There is some precedent for the existence of glutamine synthetases resistant to other compounds. It is known that methionine sulfoximime (MSO), another glutamate analogue, is a mixed competitive inhibitor ($K_i$ value of 0.16mM) of pea leaf glutamine synthetase (Leason, M., et al., Phytochemistry 21:855-857 (1982)). Miller, E.S. and Brenchley, J.E. (The Journal of Biological Chemistry 256:11307-11321 (1981)) studied the properties of several mutants of Salmonella resistant to MSO. One mutation apparently altered glutamine synthetase at the ammonia binding domain, conferring MSO resistance. More recently, Young and Ringold (ibid 258:11260-11266 (1983)) have reported that mouse 3T6 cells grown in the presence of MSO developed resistance thereto. MSO resistant cells had mRNA enriched for glutamine synthetase, and the authors suggested that this observation implied an amplification of the gene. See also Sanders and Wilson, EMBO. J., 3:65-71 (1984). Neither the Miller, Young, nor Sanders studies were reported on plant GS.

Further, prior to the present invention no studies have been reported on attempts to confer resistance to herbicidal GS inhibitors, such as PPT, by manipulating the glutamine synthetase genes in plant cells.

It would therefore be desirable to develop plant cells which are resistant to herbicidal inhibitors of GS, such as PPT, by manipulating the plant glutamine synthetase genes in said cells. In such manner, it would be impossible to confer herbicidal selectivity to any given plant.

## DISCLOSURE OF THE INVENTION

The present invention arose out of the discovery that resistance to PPT in plants can arise due to overproduction of glutamine synthetase, a phenomenon which, in the initial experiments, was shown to be due to an underlying gene amplification mechanism. Upon applying selective pressure on certain plant cells in tissue culture, it was possible to isolate PPT resistant strains. The resistance, however, was not due to the presence of a structural mutant of glutamine synthetase which had less affinity for PPT, but rather, due to gene amplification and concomitant increased concentrations of the enzyme in the plant cells. Out of these initial observations arose the concept of the invention of developing plant cells which overproduce glutamine synthetase (and thus show herbicidal GS-inhibitor resistance), either by gene amplification or by other, different, mechanisms than gene amplification.

The invention provides a recombinant DNA molecule comprising a DNA sequence coding for genomic glutamine synthetase as shown in Figure 4.

The invention also comprises intermediate vehicles capable of serving as transformation vectors for plant cells carrying genetic information as described, capable of conferring herbicidal GS inhibitor-resistance, and methods of conferring resistance to plant cells.

## BRIEF DESCRIPTION OF THE DRAWINGS

The invention will be better understood by reference to the accompanying Figures where

Figure 1 shows the glutamine synthetase/glutamate synthase cycle, wherein it is shown that GS catalyzes the formation of glutamine from glutamic acid and ammonia in a reaction driven by the hydrolysis of ATP to ADP and inorganic phosphate. The amide nitrogen of glutamine provides the source of nitrogen for many biosynthetic reactions, indicating a central role for GS in nitrogen metabolism. The herbicidal GS inhibitor, by inhibiting GS, prevents the biosynthesis of glutamine, thereby preventing ammonia detoxification.

Figure 2 shows the growth characteristics of a wild type (PPT-sensitive) alfalfa cell line in the absence (o) and in the presence of 25 (△), 50 (●) and 100 (□) $\mu$m L-PPT.

Figure 3 shows the growth characteristics of a variant PPT resistant alfalfa cell line in the absence (o) and in the presence of 100 (△), 200 (●) and 500 (□) $\mu$m L-PPT.

Figure 4 shows the complete functional genomic sequence for glutamine synthetase.

## BEST MODE OF CARRYING OUT THE INVENTION

In the description that follows, a number of terms used in recombinant DNA, plant genetics technology and in the present invention are extensively utilized. In order to provide a clear and consistent understanding of the specification and claims, including the scope to be given such terms, the following definitions are provided:

Nucleotide. A monomeric unit of DNA or RNA consisting of a sugar moiety, a phosphate, and a nitrogenous heterocyclic base. The base is linked to the sugar moiety via the glycosidic carbon (1′ carbon of the pentose). The combination of a base and a sugar is called a nucleotide. Each nucleotide is characterized by its base. The four DNA bases are adenine (A), guanine (G), cytosine (C) and thymine (T). The four RNA bases are A, G, C and uracil (U).

Genetic sequence. A linear array of nucleotides connected one to the other by phosphodiester bonds between the 3′ and 5′ carbons of adjacent pentoses.

Functional Genetic Sequence. A genetic sequence coding for a polypeptide having desired activity, regardless of whether the sequence is shorter or longer than that of the full length sequence for the polypeptide. It is also referred to as "functional gene."

Codon or Triplet. A DNA sequence of three nucleotides which encodes through mRNA an amino acid, a translation start signal or a translation termination signal. For example, Codons TTA, TTG, CTT, CTC, CTA and CTG encode for the amino acid leucine. TAG, TAA, and TGA are translation stop signals, and ATG is a translation start signal.

Exon. Those genetic sequences that are translated into amino acid sequences.

Intron. Those genetic sequences that are not translated into amino acid sequences and intervene between the exons.

Genomic sequence. The entire genetic sequence for a given polypeptide including the translated exons and the intervening untranslated introns.

Reading frame. The grouping of codons during translation of mRNA into an amino acid sequence. During translation, the proper reading frame must be maintained. For example, the sequence GCTGGTTGTAAG may be translated into three reading frames or phrases depending on whether one starts with G, with C, or with T, and thus may yield three different peptide products.

Two sequences are in operable linkage when the reading frame of one sequence is linked to the other so that both operate in combination as if they would be present independently.

Transcription. The process of producing mRNA from a functional gene.

Translation. The process of producing a polypeptide from mRNA.

Expression. The process, starting with a functional gene, to produce its polypeptide, being a combination of transcription and translation.

Cloning vehicle. A plasmid, phage DNA, or other DNA sequences which are able to replicate in a host cell, which are characterized by one or a smaller number of endonuclease recognition sites at which such DNA sequences may be cut in a determinable fashion without loss of an essential biological function of the DNA and which contain a marker suitable for use in the identification of transformed cells. A typical example is an antibiotic resistance marker. The word "vector" is sometimes used for a cloning vehicle.

Expression vehicles. A vehicle, analogous to a cloning vehicle, which is particularly useful for production in host cells of a polypeptide by expression of the functional gene coding for said polypeptide, present in the vehicle.

Replication vehicle. A cloning or expression vehicle.

Phage or bacteriophage. A bacterial virus which may consist of DNA sequences encapsulated in a protein envelope or coat.

Plasmid. A non-chromosomal double stranded DNA sequence comprising an intact "replicon," such that the plasmid is replicated in or incorporated into the genome of a host cell. When the plasmid is placed within a unicellular or multi-cellular organism, the characteristics of that organism may be changed or transformed as a result of the DNA of the plasmid. For example, a plasmid carrying the gene for kanamycin resistance transforms a cell previously sensitive to kanamycin into one which is resistant to it.

Cloning. The process of obtaining a population of organisms or DNA sequences derived from one such organism or sequence by asexual reproduction.

Expression control sequence. A genetic sequence that controls and regulates expression of functional genetic sequences when operably linked to those sequences. They include sequences known to control the regions which regulate that expression. They comprise both promoter and terminator sequences.

Plant Promotor. An expression control sequence which is capable of causing the expression in said plant of any homologous or heterologous genetic sequences or sequences operably linked to such promoter.

Overproducing Plant Promoter (OPP). A plant promoter capable of causing the expression in a transformed plant cell of any operably linked functional genetic sequence or sequences to levels (measures by mRNA or polypeptide quantities) which are substantially higher than the levels naturally observable in host cells not transformed with said OPP.

Degeneracy. An informational property according to which each amino acid in nature can be coded by more than one codon. For example, leucine can be coded by TTG, TTA, CTA, CTT, CTC or CTG.

By degenerate variations as used in the present application and claims, is meant any variation of the polynucleotide fragments of the invention due to the degeneracy of the genetic code. For example, as long as the resulting material still codes for functional GS, or portions thereof, such material is designed to be included in the present invention.

Glutamine Synthetase (GS). The definition of this enzyme is functional, and includes any glutamine synthetase capable of functioning in a given desired plant to transform glutamic acid to glutamine in the GS cycle. The term therefore includes not only the enzyme from the specific plant species involved in the genetic transformation, but may include GS from other plant species or microbes or even other eukaryotes, if such GS is capable of functioning in the transformed plant cells. The terms include proteins or polypeptides having more or less than the total structural length of natural plant GS, such as functional partial fragments of GS, or their analogues.

Phosphinothricin (PPT). The compound of formula (1), supra, in its biologically active form. It may be the L-, or D- or D,L- forms, and may be alone or in combination with other inert or active compounds which do not interfere with PPT activity.

The invention comprises at its most fundamental level a plant cell which is resistant to a herbicidal glutamine synthetase inhibitor wherein the resistance is caused by levels of GS activity which, when present in an otherwise herbicidal GS inhibitor-sensitive plant cell, render the cell substantially resistant to the herbicidal GS inhibitor.

The terms "herbicidal glutamine synthetase inhibitor" are meant to include any inhibitor, competitive or non-competitive, that significantly decreases the glutamine synthetase activity of a plant cell of a given species and, as a consequence thereof, causes herbicidal effects in the plant cell. The herbicide resistant plant cell or whole plant survives without irreversible damage a herbicidal GS inhibitor concentration which is lethal for wild type individuals of the same species. Normally, a five-fold or higher increase in herbicidal GS inhibitor resistance is considered significant. However, this number varies from plant species to plant species, and from herbicide to herbicide. It is therefore not feasible to provide a single level for all of the several plant species and herbicides covered by the present invention. Such level, however, can be readily ascertained by those skilled in the art.

Among the herbicidal GS inhibitors covered by the invention are phosphinothricin, methionine sulfoximine, as well as other glutamic acid analogues.

The glutamine synthetase may or may not be from the specific plant cell being transformed. All that is necessary is that the genetic sequence for the enzyme be expressed, and produce, a functional enzyme in the final plant cell. Thus, the invention comprises plant cells containing either homologous GS genes or heterologous GS genes (and their respective expression products). Broadly, the enzyme might also be that of other plant species, or even enzymes from different organisms, such as microorganisms or animals. Preferred are plant glutamine synthetase genes and their expression products. Of particular interest are glutamine synthetase genes from the particular plant species which serves as the host in the genetic manipulation, i.e., homologous GS genes. One such glutamine synthetase gene utilizable in the rDNA molecules of the invention is illustrated in Example 2.

Any plant cell which is sensitive to herbicidal GS inhibitors, which is capable of undergoing genetic manipulation by the genetic constructs or methods of the invention, and is capable of expressing said constructs, can be used in the present invention. Among dicotyledonous plants are included various species of potato (Solanum tuberosum); tomato (Lycopersicon esculentum); pepper (Capsicum anumm); tobacco (Nicotiana tabacum); various species of Brassica, especially rapeseed (Brassica napus), various legumes, for example alfalfa (Medicago sativa), clover (Trifolium spec.), soybean (Glycine max), grondnut (Arachis hypogaea), various species of beans (Phaseolus spec., Vicia spec., Vigna spec.), peas (Pisum sativum), root crops as beets (Beta vulgaris), carrots (Daucus carota) and sweet potatoes (Ipomoea batatus).

There are a variety of embodiments encompassed in the broad concept of the invention. The herbicidal GS-inhibitor resistant plant cell can contain significantly higher levels of GS activity by any of a variety of mechanisms and/or genetic constructs.

For example, the invention comprises, in one of its embodiments, a combination of two genetic sequences: (a) a first genetic sequence coding for a genomic glutamine synthetase functional in a given plant cell, operably linked downstream from (b) a second genetic sequence capable of increasing the levels of gene product of said first sequence such that when the combination is present in an otherwise herbicidal GS-inhibitor sensitive plant cell, said cell is substantially resistant to said herbicidal GS-inhibitor.

The second genetic sequence may be a promoter or an enhancer sequence. If a promoter, it may be a GS promoter or a promoter of another structural gene. In either of the latter two events, the promoter useful in the combination is an overproducing plant promoter. The only essential characteristic of such promoter is that, when in operably linkage with the genetic sequence for glutamine synthetase, it be capable of promoting expression of said glutamine synthetase to levels such that when the combination is present in an otherwise herbicidal GS-inhibitor sensitive plant cell, the cell is substantially resistant to said GS-inhibitor. Thus, the choice of what plant promoter to use is ruled by functional considerations. The plant promoter will be heterologous or homologous to the host cell. The native, endogenous promoter of said host cell would be incapable of causing resistance by overproduction of GS, since such native promoter normally promotes expression of GS to levels which are so low as to be substantially or completely inhibited by normally used plant-controlling herbicide concentrations. Thus, under one embodiment of the present invention, the native promoter is replaced by an OPP.

Among useful OPP's are included the promoter of the small subunit (ss) of the ribulose bi-phosphate carboxylase, and of the chlorophyll a/b binding protein. The expression of these two genes has been shown to be light induced at the transcriptional level in green tissue (see, for example, Genetic Engineering of Plants, An Agricultural Perspective, A. Cashmore, Plenum, New York 1983, pages 29-38; Coruzzi, G., et al., The Journal of Biological Chemistry 258:1399 (1983); or Dunsmuir, P., et al., Journal of Molecular and Applied Genetics, 2:285 (1983)).

The invention extends to any plant cell modified according to the methods described, or modified by any other methods which yield herbicidal GS-inhibitor resistance. The plant cell may be alive or not, by itself, in tissue culture, or as part of a multicellular plant or a part thereof. Such a multicellular plant, which in its untransformed state is herbicidal GS-inhibitor sensitive, would be resistant when its cells are resistant

according to the invention.

Parts obtained from the plant, such as flowers, seeds, leaves, branches, fruit, and the like are also covered by the invention, as long as these parts comprise herbicidal GS-inhibitor resistant cells, as noted. In particular, plant parts may be alive or not. Thus, a genetically modified tomato, carrot, or tobacco leaf obtained from a resistant tomato, carrot or tobacco plant is included in the invention, even if separate from the plant of origin.

PROCESSES OF PRODUCTION AND INTERMEDIATES USED THEREIN

Various methodologies are applicable to carry out the different embodiments of the invention. For example, if the herbicidal GS-inhibitor resistance is brought about by providing significantly increased copies of a structural GS gene, available methodologies include selection for a cell which is resistant by virtue of GS gene amplification, or, alternatively, introduction of multiple copies of the GS gene into the genome or into a replicating extrachromosomal element.

Selection is carried out in an appropriate culture medium by cultivating plant cells in the presence of stepwise increases of the herbicidal GS-inhibitor in the medium. After a given period of time has elapsed, such as, for example, a few weeks to several months, it is possible to select a cell population which is several-fold more resistant to the herbicidal GS-inhibitor than the original plant cells. For example, when the cells are alfalfa and the inhibitor is PPT, it has been possible to obtain a PPT-resistant alfalfa cell line after one year of stepwise PPT increases, which line was 20-fold more resistant to PPT than the original line. When the resistant line was subcultured for several months in the absence of the inhibitor, a slow decline in the percentage of resistant cells was observed in plating experiments on inhibitor-containing agar media, but after more than six months it was still possible to reselect highly resistant cell clones. This was never possible by plating wild-type cells.

Regeneration of calli and grown plant from tissue culture cells is known to those skilled in the art, and it varies from species to species. See, for example, Sheperd, Scientific American, 1982. Generally, a suspension of protoplasts containing multiple copies of the GS gene or containing the genetic sequence combination is first provided. Embryo formation can then be induced from the protoplast suspensions, to the stage of ripening the germination as natural embryos. The culture media will generally contain various amino acids, and hormones such as auxin and cytokinins. It is advantageous to add glutamic acid and proline to the medium, especially for such species as corn and alfalfa. Shoots and roots normally develop simultaneously. Efficient regeneration will depend on the medium, on the genotype, and on the history of the culture. If these three variables are controlled, then regeneration is fully reproducible and repeatable.

For the genotypes of some species, such as tobacco, alfalfa, potato, tomato, petunias, soy beans, rapeseed, and some fruit trees and carrots, regeneration has been clearly demonstrated in the prior art. Such regeneration is well within the skill of the art if the appropriate genotype and history is chosen by screening. Generally, after about more than one year of subculturing, the efficiency of plant regeneration decreases from a given cell culture. Therefore, regeneration is normally most successful if it is initiated shortly after the provision of an appropriate resistant cell culture.

Alternatively, and especially useful in cell cultures which are older than several months or one year, the amplified GS gene can be carried out into a regenerative system by crossing and/or fusion. See, for example, Cocking et al., Nature, 293:265 (1981). In this method, a herbicidal GS-inhibitor resistant cell line is provided in selection, and a culture suspension of rapidly dividing cells is obtained by methods well known to those of skill in the art. Protoplasts are isolated therefrom, and preferably irradiated with an amount of radiation and for a time sufficient to inactivate, if not completely irreversibly damage, the host cells. For example, X ray radiation of 20 Krads can be used advantageously. After irradiating the donor protoplasts, the same are fused with acceptor protoplasts from appropriate susceptible cells. Various methods exist to obtain fusions, such as, for example, polyethylene glycol fusion, high calcium treatment, combinations of both, or even recently, electrofusion. After fusion has occurred, selective growth of the fusion product can be carried out since the fusion product should be herbicidal GS-inhibitor resistant. In particular, if a donor is used which is no longer morphogenetic (i.e., the history of the donor culture is such that several months or upwards of a year may have already elapsed since the original selection), then a simple selection is herbicidal GS-inhibitor containing media can be carried out.

Another method of introducing genetic material into plant cells is to infect a wounded leaf of the plant with transformed A. tumefaciens bacteria. Under appropriate growth conditions a ring of calli forms around the wound. The calli are then transferred to growth medium, allowed to form shoots, roots, and develop further into plants. Alternatively, grafting onto whole plants can also be done.

An alternative method to introduce multiple copies of the GS gene into the plant cell is by self-ligating a copy of a functional GS (genomic or cDNA) structural gene, utilizing methods well known to those of skill in recombinant DNA technology. The structural genes, each containing its individual promoter, are operably linked to each other by means of appropriate linkers, and 10-30 copies of each gene can in certain instances be introduced into an appropriate replicable expression vehicle, such as a Ti plasmid. Alternatively, the genetic material can be micro-injected directly into plant embryo cells. In the case of monocotyledonous plants, pollen may be transformed with total DNA or an appropriate functional clone providing resistance, and the pollen then utilized to produce progeny by sexual reproduction.

Of course, any other methods utilizable to increase GS activity in a given cell to such levels as will make the cell herbicidal GS-inhibitor resistant can be utilized. Of particular interest in this invention is the operable linkage of a functional genomic sequence coding for a structural GS gene to another genetic sequence capable of overproduction of the gene product derived therefrom. This linkage of genetic sequences can be introduced into appropriate plant cells, for example, by means of the Ti plasmid.

The introduction of genetic material into plant cells, especially by use of the so-called tumor inducing (Ti) plasmid of Agrobacterium tumefaciens, is a reproducible and predictable technology. (See, for example, Caplan, A., et al., "Introduction of Genetic Material into Plant Cells," Science: 815-821 (November 1983); Schell, J., and Van Montagu, M., "The Ti Plasmid as Natural and as Practical Gene Vector for Plants," Bio/Technology: April 1983, pp. 175-180; Horsch et al., "Inheritance of Functional Foreign Genes in Plants," Science: 233, 496-498 (1984); Fraley, R.T., et al., Proc. Natl. Acad. Sci. USA 80:4803 (1983); Watson et al., Recombinant DNA. A Short Course, Scientific American Books, 1983, pp. 164-173; and Old and Primrose, Principles of Gene Manipulation, 2d Ed., U. Cal. Press, 1981, pp. 138-156.)

Ti plasmids contain two regions essential for the production of transformed cells. One of these, named transfer DNA (T DNA), induces tumor formation. The other, termed virulent region, is essential for the formation but not maintenance of tumors. Transfer DNA, which transfers to the plant genome, can be increased in size by the insertion of the multiply linked GS genes or of the gene combination of the invention without its transferring ability being affected. By removing the tumor causing genes so that they no longer interfere, the modified Ti plasmid can then be used as a vector for the transfer of the gene constructs of the invention into an appropriate plant cell. The foreign DNA to be inserted is usually introduced between the terminal sequences flanking the T-region.

A particularly useful Ti plasmid vector is pGV3850, a non-oncogenic derivative of the nopaline Ti plasmid C58. (See Caplan et al., supra.) This vector utilizes the natural transfer properties of the Ti plasmid. The internal T-DNA genes that determine the undifferentiated crown gall phenotype have been deleted and are replaced by any commonly used cloning vehicle (such as pBR 322). The cloning vehicle sequence contained between T-DNA border regions serves as a region of homology for recombination to reintroduce foreign DNA cloned in a derivative of the same cloning vehicle. Any gene construct of the invention cloned in such plasmid can thus be inserted into pGV 3850 by a single recombination of the homologous sequences. Antibiotic resistance markers can be added to the plasmid to select for the recombination event. Herbicide resistance can of course be also used concomitantly or independently. The presence of the nopaline synthase (nos) gene in this vector makes it easy to monitor the efficiency of transformation using pGV 3850. A callus in tissue culture can then be tested for the presence of nopaline.

After transformation of the plant cell or plant, the same may be selected by aid of an appropriate marker, such as antibiotic resistance, or more relevant, herbicide resistance, and then grown in conventional ways. In tobacco, protoplast cultures three to five days after the protoplast isolation are suitable for the transformation by Agrobacteria harboring the appropriate Ti-plasmid which contains in its T-DNA region the hybrid GS-gene described. After two days of cocultivation of the protoplast borne cells and the Agrobacteria, the plant cells can be washed by centrifugation and resuspended on fresh medium several times. This removes most of the bacteria. The remaining bacteria are killed by a suitable antibotic, for example cefotaxime (400-1,000 μg/ml), added to the protoplast culture medium. The cells are cultivated on nonselective media until they form visible cell aggregates (calli). Then they are plated on media containing the proper herbicide concentration which kills all wild-type cells. Only the transformants which express the incorporated GS-hybrid gene survive and continue to grow. These calli can be easily induced to regenerate shoots when transferred to Murashige and Shoog-medium containing 1 mg/l 6-benzyladenine and 0.1 mg/l naphthalene acetic acid. The shoots can be rooted on hormone-free MS-medium or directly on perlite or vermiculite and transferred to pots. The plantlets are ready to grow in the greenhouse and can be tested for herbicide resistance.

Other systems, such as cauliflower mosaic virus, CaMV (Hohn, B., et al., in "Molecular Biology of Plant Tumors," Academic Press, New York, 1982, pp. 549-560; and Howell, United States Patent 4,407,956) can als be used. The entire CaMV viral DNA genome is inserted into a parent bacterial plasmid creating a

recombinant DNA molecule which can be propagated in bacteria. After cloning, the recombinant plasmid is cleaved with restriction enzymes either at random or at unique sites in the viral portion of the recombinant plasmid for insertion of the gene combination of the invention. A small oligonucleotide, described as a linker, having a unique restriction site may also be inserted. The modified recombinant plasmid again may be cloned and further modified by introduction of larger pieces of a gene construct into the unique restriction site of the linker. The modified viral portion of the recombinant plasmid is then excised from the parent bacterial plasmid and used to innoculate the plant cells or plants. This virus is described in the aforementioned Howell patent as being particularly good for insertion of genes capable of enhanced production of protein, greater tolerance to stress, resistance to pests and pesticides, nitrogen fixation, and the like.

Normally, the desired GS sequences are operably linked in vitro to each other or to an overproducing promoter, by known recombinant methodology. For example, the structural gene for GS, normally the genomic version thereof, in separated from its normal promoter by restriction on the region between such promoter and the initiation AUG codon. A transcriptional fusion with, e.g., the small subunit of the ribulose bi-phosphate carboxylase is then carried out. The construct is then inserted into an appropriate restriction site of the plant vehicle.

For example, when using CaMV DNA as a vehicle, the genetic construct is inserted into a site or sites in the viral DNA, without destroying infectivity of the viral DNA or its movement throughout the plant. Thus, the construct can be inserted into a variety of restriction sites, cloning the product and determining whether the essential characteristics of the virus have been retained. In this manner, one can rapidly isolate a relatively large amount of modified virus which can be screened for infectivity and movement. After the viral portion of the hybrid DNA plasmid has been modified, the modified virus may be excised from the hybrid DNA plasmid, and may be used to inoculate plants directly in linear form or ligated in circles.

Various techniques may be employed for infecting plant cells with CaMV vehicles. Young leaves may be mildly abraded and then contacted with the viral DNA. After infection, the viral DNA may be transmitted by aphids, where the aphid transmissible gene is operative. Mechanical techniques can also be employed. Alternatively, tissues or single cells may be infected.

## USES

The use of vehicles containing the gene constructs of the invention is as intermediates in the preparation of whole herbicide-resistant plant cells and plants. Thus, not only the plant cells made resistant according to the methods of the invention, but also all of the vehicles or vectors, derive their utility from the utility of the final product, the whole plant.

The utility for a whole plant made herbicide resistant according to the invention is obvious. Such a plant, when brought into contact with otherwise plant controlling or suppressing amounts of herbicide, would be resistant thereto. This would allow herbicide treatment to be selective for any desired plant or groups of plants.

In addition, the resistance to herbicide would enable its use as a selectable marker in the transfer of other genes to the plant, or cells thereof.

By the term "plant controlling amounts of herbicidal GS-inhibitor" is meant to include functionally an amount of herbicide which is capable of affecting the growth or development of a given plant. Thus, the amount may be small enough to simply retard or suppress the growth or development, or the amount may be large enough to irreversibly destroy the sensitive plant. Normally, most dicotyledonous plants and weeds may be controlled at rates of between 0.5 to 1.5 kg/ha ai. For monocotyledonous plants, rates between 0.5 kg/ha ai, and up to about 2.0 kg/ha ai are normally used. The herbicide can, of course, be contacted with the appropriate plant using well-known spraying or spreading methods. For example, foliar administration used in the prior art for control of weeds by PPT can be used with PPT-resistant plants falling within the invention.

The invention also encompasses a method of plant control which comprises contacting a herbicidal GS-inhibitor sensitive plant cell or plant, with plant controlling amounts of herbicidal GS-inhibitor, wherein the contact is carried out while the sensitive plant cell or plant is present simultaneously with or among the herbicidal GS-inhibitor resistant plant cells or plants of the invention. Thus, foliar herbicidal treatment of plants in a field or cultivar, which plants include both those comprising herbicide resistant plant cells and those comprising herbicide sensitive plant cells, and wherein both are simultaneously contacted with the herbicidal GS inhibitor during the treatment operation, is a method included in the present invention.

Having now generally described this invention, the same will become better understood by reference to certain specific examples which are included herein for purposes of illustration only and are not intended to

be limiting unless otherwise specified.

Example 1

Isolation and Characterization of PPT-Resistant Alfalfa Cell Culture

An alfalfa cell line which is able to grow in the presence of 3 mM L-phosphinothricin in the culture medium was selected. Alfalfa was chosen since it is one of the model plants in which the regeneration of a whole plant from single isolated cells or protoplasts can be readily achieved.

The selection of the phosphinothricin resistant alfalfa line was done by stepwise increase of the inhibitor concentration in the liquid culture medium. Within six months a cell population was selected which was at least 20-fold more resistant to phosphinothricin than the original cell line. Measuring the packed cell volume of the suspension cultures revealed that wild-type cells were completely inhibited by 2.5 x $10^{-5}$ M L-phosphinothricin. The resistant cells grew as well in the presence of 5 x $10^{-4}$ M of the compound as in its absence (Figures 2 and 3).

When the protein patterns of crude cell extracts of the susceptible and resistant cell lines were compared by SDS-polyacrylamide gel electrophoresis, the overproduction of a polypeptide in the molecular weight range 40-42 KD could be observed in the PPT-resistant cell line. Purified GS of alfalfa has the same molecular weight. The enzyme activity of the cell extracts was determined in parallel. The specific GS-activity in the resistant cells was 5-10 fold higher than in wild-type cells. The specific activity of GS in the PPT-resistant cell lines is in the same order of magnitude as in root nodule tissue of alfalfa, which, as all nodule tissues of efficiently dinitrogen fixing legumes, has high GS-activity. Measurements of the GS activity in the presence of various amounts of phosphinothricin revealed the same degree of enzyme inhibition by PPT in both cell types, suggesting that no structural change of the GS-protein had occurred during the selection of the resistant variant.

Messenger RNA was isolated from wild-type and resistant cells using a guanidinium isothiocyanate RNA-extraction method, followed by the separation of the mRNA through an oligo dT-cellulose column. Four microgram mRNA could be isolated per gram of cell material. In vitro translation of the isolated mRNA from both cell lines yielded patterns of polypeptides similar to the in vivo patterned obtained in $^{35}$S-methionine labeled extracts. This indicated that the isolated mRNA was fairly undegraded. While the in vivo labeled proteins showed a striking difference in the amount of the 40-42 KD polypeptide, the difference between wild-type the variant cell line in the in vitro translated protein patterns was not pronounced.

First and second strand cDNA were synthesized from the mRNA population of the PPT-resistant variant. After $S_1$-cleavage and poly C-tailing of the cDNA, the DNA was annealed to poly G-tailed pBR 322 vector DnA. E. coli strain MC1061 was transformed with the recircularized vector.

3.5 x $10^4$ colonies were obtained per mg of annealed vector DNA. 80% of the colonies were ampicillin (amp) sensitive, and 50% of the amp sensitive clones had inserts which could be detected by Pst digestion of the plasmids. 10% of the inserts were longer than 1,000 base pairs.

1,800 colonies were grown on nitrocellulose filters and the filters were probed with $^{32}$p-labeled first strand cDNA probe having increased percentage of GS-specific sequences. Amongst such clones it was expected to find a GS-specific cDNA clone. A GS-cDNA clone from a Phaseolus cDNA library prepared from mRNA of root nodules was obtained from an outside source. This GS-cDNA clone enabled the identification of one alfalfa cDNA clone in the cDNA library, which hybridized strongly to the Phaseolus GS-DNA.

The insert DNA of the alfalfa GS-cDNA clone was sequenced. The alfalfa and the Phaseolus GS-cDNAs were then used as probes to characterize the difference between the wild alfalfa cells and the variant, GS-overproducing cells.

Total genomic DNA was prepared from alfalfa leaves, from wild-type cell line and from three sublines of the resistant cell line which had been selected in presence of 6 x $10^{-4}$ M, 2 x $10^{-3}$ M, and 3 x $10^{-3}$ M L-phosphinothricin. The DNA was digested with Bam HI, Eco RI, and HindIII, and a combination of two enzymes. After agarose gel electrophoresis of the digested DNAs, the DNA fragments were transferred onto nitrocellulose filters by Southern blotting. The filter-bound DNA was hybridized with $^{32}$P-labeled cDNA inserts of the alfalfa and the Phaseolus GS-clones. DNA of the same size hybridized with both probes. One predominant band hybridizes with the same intensity in all five DNA samples with the alfalfa probe. A second band, which is barely visible in the wild-type DNA digest, strongly hybridizes with DNA of the resistant cell lines. The highest degree of hybridization was observed with DNA of the highly resistant cell lines, indicating amplification of a DNA fragment during the development of the resistance. The alfalfa cDNA probe strongly hybridized to the amplified DNA fragment and, to a lesser extent, to the amplified DNA with

GS homology.

In order to confirm the reason why the variant alfalfa cell line had become resistant to PPT, both Northern and Southern blots were repeated using wild-type and variant cell lines. The Northern blot indicated that there is an increase of about 8-fold in glutamine synthetase mRNA from wild-type. When Southern blots were done using genomic DNA from both cell lines, there was a clear indication of gene duplication of glutamine synthetase in the mutant alfalfa cell line. The duplication seems to be 5- to 15-fold above the unduplicated glutamine synthetase gene as estimated by hybridization. It also appeared to be an exact duplication, that is, only one band increased in hybridization by 7- to 11- fold.

Example 2

Isolation and Characterization of Glutamine Synthetase from the PPT-Resistant Cell Culture

**Materials and Methods**

CNBr Cleavage

Glutamine synthetase (12 nmol) was dissolved in 1mL of 70% formic acid, 5 mg CNBr was added, and the cleavage proceeded for 24 hr at room temperature (Gross, Meth. Enzymol 11:238-255 (1967)). After dilution with distilled water (6 mL), the mixture was lyophilized twice.

High Performance Liquid Chromatography of CNBr Peptides

Reverse-phase high performance liquid chromatography (HPLC) was carried out on an Ultrapore RPSC (0.46 ID x 7.5 cm) at 40 °C using 0.1% trifluoroacetic acid (TFA) (Bennet et al., Biochem. J. 168:9-13 (1977)) and a linear gradient of 0-60% acetonitrile (30 min; flow rate, 0.5 mL/min). The CNBr peptides were dissolved in the minimum volume of 6 M guanidine hydrochloride in 0.1% TFA. Chromatographic peaks from several separations were collected manually, pooled, and lyophilized. Detection was at 214 nm using a Beckman 160 detector.

Amino Acid Analysis

Amino acid compositions were determined after acid hydrolysis of samples in sealed, evacuated tubes (Pyrex®, culture, rimless, 6x50 mm) at 110 °C for 24 hr in constant boiling HCl (0.025 mL) (Moore, Chemistry and Biology of Peptides, Ann Arbor Science, Ann Arbor, Michigan, 629-653 (1972)). Using a Beckman 6300 amino acid analyzer equipped with two Hewlett-Packard 3390A integrators, ninhydrin and two channel (440 and 570 nm) integration provided analysis of all amino acids, except tryptophan, cysteine, asparagine, and glutamine, with confidence values of 1-7% at 100 pmol/amino acid, and with a lower limit of detection of 25 pmol. Analyses were done 3-5 times on each sample and background controls.

Protein sequence analysis:

Automated Edman degradation was performed with an Applied Biosystems 470A sequencer. The sequence program was developed earlier for the gas-liquid solid phase peptide and protein sequencer (Hewick et al., J. Biol. Chem., 256:7790-7997 (1981)). The program contains one coupling step (44 °C, 26 min) and a single cleavage step (44 °C, 6.7 min). Automated conversion of the 2-anilino-5-thiazolinone derivatives (Pth-amino acids) uses 25% trifluoroacetic acid (50 C, 33 min). Polybrene® (1.5 mg) (Tarr et al., Anal. Biochem. 84:622-627 (1978); Klapper et al., ibid, 85:126-131 (1978)) was added to the glass filter disc in the cartridge prior to degradation of protein or peptides, and five sequence cycles were run to reduce contaminants derived from Polybrene®. Angiotensin II (1 nmol) was added to the cartridge filter, and then degradative cycles were completed. Sequencing of angiotensin allowed an assessment of the chemical and mechanical operation of the sequencer prior to the sequencing of the unknown. Protein sequence analysis of sperm whale apomyoglobin (100-200 pmol) routinely demonstrated an initiated yield of 45-55%, average repetitive yield of 92-93%, and an average lag per cycle of 2-3%. All Pth-amino acids were identified by reverse-phase high performance liqud chromatography on a cyano column (0.45 x 25 cm) using a 15 mM sodium acetate buffer (pH 5.5), and a complex gradient of acetonitrile/ methanol (92.5:7.5, v/v), based on a system developed by Hunkapiller and Hood, Methods Enzymol. 91:486-493 (1983). This HPLC system separated the Pth-amino acids and internal standard (methyl ester of Pth-Glu) from the major contaminants:

a dithiothreitol-adduct, N-dimethyl-N[1]-phenylthiourea, and diphenylthiourea. Methionine and proline were not routinely separated, but a modification of the gradient separated these two Pth-amino acids. Internal standard (200 pmol) was added to each cycle collection, and the samples were dried in a Speedvac Concentrator with an RH100-6 rotor. The dried samples were dissolved in 0.025 mL of water/acetonitrile (80:20 v/v), and an aliquot (0.017 mL) (68% of total sample) injected automatically. The Pth-amino acids were detected by UV absorbance at 254 nm using a Beckman 160 detector.

Purification Scheme

The process of purification is briefly summarized in the following scheme:

```
100 gm. frozen cells
    Add 50 mM Tris-pH 7.5
    Use blender low speed (30 seconds)
                high speed (2 minutes)
    Spin 8K rpm, 5°, 10 minutes
Supernatant     7.5% v/v 1% protamine sulfate,
                15 minutes, 4°
    Spin 8K rpm, 5°, 10 minutes
Supernatant     1.5% v/v 1 M MgSO4
                pH to 6.8 with 1 N acetic acid
                β-mercaptoethanol to 7 mM
Bind to hydroxyapatite, 4°, 15 minutes
Wash hydroxyapatite  5x with 50 mM Tris pH 7.5
                     0.28 M MgSO4
                     10% ethylene glycol
Elute GS 5x with 50 mM Tris pH 7.5
                 0.5 M MgSO4
                 16.7% ethylene glycol
Pool eluted fractions, dialyze against 10 mM Tris
                                       pH 6.8
                                       10 mM Mg
                                       MgSO4
Sephadex G-200 column, pool activity peak
```

100 grams of frozen variant alfalfa tissue culture cells were mixed with dry ice and ground to a fine powder in a blender. After thawing, the mixture was spun at 8,000 rpm at 4° for 20 minutes. Protamine sulfate precipitation was carried out on the supernatant to remove nucleic acid. The $MgSO_4$ concentration

was brought to 10 mM, the β-mercaptoethanol (BMe) to 7 mM, and the pH adjusted to 6.8 with 1N acetic acid. The crude extract was bound to hydroxyapatite (H.A.). The H.A. was washed batchwise five times with 10 mM Tris, pH 7.5, 0.28 M $MgSO_4$, and 10% ethylene glycol. Glutamine synthetase was eluted by bringing the $MgSO_4$ concentration to 0.5 M. Five batch elutions were done. After dialyzing against 10 mM Tris, pH 7.5, 10 mM $MgSO_4$, it was loaded onto a Sephadex G-200 column. Fractions with good glutamine synthetase activity were pooled. By SDS acrylamide gel and silver staining, the protein was better than 95% pure. An amino acid composition showed the composition of alfalfa glutamine synthetase to be nearly the same as that of a published composition from soy bean (Table 1).

**Table 1**

**Amino Acid Composition of Alfalfa Glutamine Synthetase**

| Amino Acid | Soy Bean* Root (mole %) | Pea Leaf (mole %) | Alfalfa (mole % + C.V.) |
|---|---|---|---|
| Ala | 8.4 | 6.9 | 8.7 (±0.4) |
| Arg | 5.6 | 4.0 | 5.0 (±0.5) |
| Asx | 10.4 | 10.7 | 11.4 (±0.6) |
| Cys | 0.8 | N.D. | N.D. |
| Glx | 10.0 | 10.1 | 9.9 (±0.5) |
| Gly | 10.6 | 19.1 | 13.5 (±0.7) |
| His | 2.2 | 3.6 | 3.1 |
| Ile | 6.6 | 5.6 | 8.4 (±0.4) |
| Leu | 6.7 | 6.5 | 7.6 (±0.9) |
| Lys | 5.9 | 5.7 | 6.8 (±0.3) |
| Met | 1.6 | N.D. | 0.6 (±0.3) |
| Phe | 2.8 | 2.6 | 2.3 |
| Pro | 6.1 | 8.8 | 8.0 (±0.6) |
| Ser | 5.4 | 7.8 | 6.5 (±0.4) |
| Thr | 5.1 | 4.9 | 5.6 (±0.3) |
| Trp | 1.5 | N.D. | N.D. |
| Tyr | 3.9 | 0.5 | 3.4 (±0.7) |
| Val | 6.3 | 4.8 | 4.4 (±1.5) |

*R.H. McParland *et al.*, *Biochem. J.* (1976) **153**:597-606.
N.D. = Not Determined.
C.V. = Confidence Value (S.D./mean)

Sequencing

A first attempt at sequencing was initiated using the purified native protein. There was no obtainable sequence, indicating that the $NH_2$-terminal was naturally blocked. A CNBr cleavage was carried out, and the fragments were separated by HPLC. The amino acid composition of the protein indicated that there were only two to five methionines. Two of the HPLC separated CNBr fragments yielded useful sequence information.

Sequencing of purified glutamine synthetase fragment was then carried out. The results are as follows:

Arg-Glu-Asp-Gly-Gly-Tyr-Glu-Val-Ile-
Leu-Lys-Ala-Ile-Glu-Lys-Leu-Gly-Lys
Lys-(Glu/His)-Lys-Glu-His-Ile-Ala-
Ala-Tyr-Gly-Gly-Gly-Asn

This sequence corresponds to part of the sequence obtained from the complete variant alfalfa cDNA clone with the exception of the circled Gly residue. The deduced protein sequence is shown on the following page.

```
   1 ATG TCT CTC CTT TCA GAT CTT ATC AAC CTT GAC CTC TCC GAA ACC ACC GAG AAA ATC ATC GCC GAA TAC ATA TGG   75
     Met Ser Leu Leu Ser Asp Leu Ile Asn Leu Asp Leu Ser Glu Thr Thr Glu Lys Ile Ile Ala Glu Tyr Ile Trp

  76 ATT GGT GGA TCT GGT TTG GAC TTG AGG AGC AAA GCA AGG ACT CTA CCA GGA CCA GTT ACT GAC CCT TCA CAG CTT  150
     Ile Gly Gly Ser Gly Leu Asp Leu Arg Ser Lys Ala Arg Thr Leu Pro Gly Pro Val Thr Asp Pro Ser Gln Leu

 151 CCC AAG TGG AAC TAT GAT GGT TCC AGC ACA GGT CAA GCT CCT GGA GAA GAT AGT GAA GTT ATT ATC TAC CCA CAA  225
     Pro Lys Trp Asn Tyr Asp Gly Ser Ser Thr Gly Gln Ala Pro Gly Glu Asp Ser Glu Val Ile Ile Tyr Pro Gln

 226 GCC ATT TTC AAG GAC CCA TTT AGA AGG GGT AAC AAT ATC TTG GTT ATG TGT GAT GCA TAC ACT CCA GCT GGA GAG  300
     Ala Ile Phe Lys Asp Pro Phe Arg Arg Gly Asn Asn Ile Leu Val Met Cys Asp Ala Tyr Thr Pro Ala Gly Glu

 301 CCC ATT CCC ACC AAC AAG AGA CAT GCA GCT GCC AAG ATT TTC AGC CAT CCT GAT GTT GTT GCT GAA GTA CCA TGG  375
     Pro Ile Pro Thr Asn Lys Arg His Ala Ala Ala Lys Ile Phe Ser His Pro Asp Val Val Ala Glu Val Pro Trp

 376 TAT GGT ATT GAG CAA GAA TAC ACC TTG TTG CAG AAA GAC ATC AAT TGG CCT CTT GGT TGG CCA GTT GGT GGT TTT  450
     Tyr Gly Ile Glu Gln Glu Tyr Thr Leu Leu Gln Lys Asp Ile Asn Trp Pro Leu Gly Trp Pro Val Gly Gly Phe

 451 CCT GGA CCT CAG GGA CCA TAC TAT TGT GGA GCT GGT GCT GAC AAG GCA TTT GGC CGT GAC ATT GTT GAC TCA CAT  525
     Pro Gly Pro Gln Gly Pro Tyr Tyr Cys Gly Ala Gly Ala Asp Lys Ala Phe Gly Arg Asp Ile Val Asp Ser His

 526 TAC AAA GCC TGT CTT TAT GCC GGC ATC AAC ATC AGT GGA ATC AAT GGT GAA GTG ATG CCT GGT CAA TGG GAA TTC  600
     Tyr Lys Ala Cys Leu Tyr Ala Gly Ile Asn Ile Ser Gly Ile Asn Gly Glu Val Met Pro Gly Gln Trp Glu Phe

 601 CAA GTT GGT CCC TCA GTT GGT ATC TCT GCT GGT GAT GAG ATA TGG GTT GCT CGT TAC ATT TTG GAG AGG ATC ACT  675
     Gln Val Gly Pro Ser Val Gly Ile Ser Ala Gly Asp Glu Ile Trp Val Ala Arg Tyr Ile Leu Glu Arg Ile Thr

 676 GAG GTT GCT GGT GTG GTG CTT TCC TTT GAC CCA AAA CCA ATT AAG GGT GAT TGG AAT GGT GCT GGT GCT CAC ACA  750
     Glu Val Ala Gly Val Val Leu Ser Phe Asp Pro Lys Pro Ile Lys Gly Asp Trp Asn Gly Ala Gly Ala His Thr

 751 AAT TAC AGC ACC AAG TCT ATG AGA GAA GAT GGT GGC TAT GAA GTC ATC TTG AAA GCA ATT GAG AAG CTT GGG AAG  825
     Asn Tyr Ser Thr Lys Ser Met Arg Glu Asp Gly Gly Tyr Glu Val Ile Leu Lys Ala Ile Glu Lys Leu Gly Lys

 826 AAG CAC AAG GAG CAC ATT GCT GCT TAT GGA GAA GGC AAC GAG CGT AGA TTG ACA GGG CGA CAT GAG ACA GCT GAC  900
     Lys His Lys Glu His Ile Ala Ala Tyr Gly Glu Gly Asn Glu Arg Arg Leu Thr Gly Arg His Glu Thr Ala Asp

 901 ATT AAC ACC TTC TTA TGG GGT GTT GCA AAC CGT GGT GCG TCG ATT AGA GTT GGA AGG GAC ACA GAG AAA GCA GGG  975
     Ile Asn Thr Phe Leu Trp Gly Val Ala Asn Arg Gly Ala Ser Ile Arg Val Gly Arg Asp Thr Glu Lys Ala Gly

 976 AAA GGT TAT TTC GAG GAT AGG AGG CCA TCA TCT AAC ATG GAT CCA TAT GTT GTT ACT TCC ATG ATT GCA GAC ACC 1050
     Lys Gly Tyr Phe Glu Asp Arg Arg Pro Ser Ser Asn Met Asp Pro Tyr Val Val Thr Ser Met Ile Ala Asp Thr

1051 ACC ATT CTC TGG AAA CCA TAA   1071
     Thr Ile Leu Trp Lys Pro End
```

Preparation of a Functional Genomic Sequence Coding for Glutamine Synthetase

Total genomic DNA was obtained from the alfalfa tissue culture using a protocol similar to that used to obtain that of Phaseolus root nodules (Cullimore, J.D., and Miflin, B.J., FEBS Letters 158:107-112 (1983)), and digested totally with Bam HI. The material was then size fractionated in a potassium acetate 5-20% gradient and separated into aliquots. Each aliquot was then run on a 1% agarose gel and probed in a

Southern transfer system with cDNA glutamine synthetase probe obtained from a Pst cut of the coding sequence probe shown above. Positively hybridizing fractions, selected for fragments of size greater than or equal to 4 Kb, were ligated to a BV-2 lambda vector. 100,000 phages were plated and probed with the glutamine synthetase cDNA probe as above. Four positive plaques were obtained. These were then grown and plaque-hybridization purified for 3-4 rounds. A purified 4 Kb genomic clone was isolated and religated into M13mp9. The clone was sequenced from both ends. In addition, the clone was also fragmented with Hae III, subcloned into M13mp9 and again sequenced.

In this manner, this 4 Kb 5′ fragments (as well as part of an 8 Kb 3′ fragment also obtained from the digestion of the genomic DNA) was completely sequenced. Both of these fragments together provided a complete functional gene, the reading frame being predicted from the protein sequence information at hand, the known GS molecular weight and the relative incidence of stop triplets in the three possible reading frames. The complete genomic sequence for glutamine synthetase, including intron and exon regions, is shown in Figure 4. With a complete functional sequence for GS at hand, the same can be expressed by any of the methods described previously.

Figure 4 shows that there are thirteen introns in the complete genomic sequence. There is one intron before the first exon, one intron after the last exon and eleven introns between the twelve exons. Hereinafter, the introns will be labelled $I^1$-$I^{13}$ to describe their position within the genomic sequence. When such an intron is located between the first and second nucleotides of a codon or triplet, the amino acid corresponding to that triplet will be written as T-$I^x$-yr, for Tyrosine, for example. When an intron is located between the second and third nucleotides of the codon, the corresponding amino acid will be written as Ty-$I^x$-r, for example.

Introns may be of varying lengths; both the minimum and maximum lengths are determined by steric constraints. There must be enough nucleotides in the intron to allow proper looping out of the intron and proper cutting and linking of the exons. For example, in Figure 4, the largest intron is $I^1$ which is located before the first exon and is 740 nucleotides long.

Having now fully described this invention, it will be understood that the same can be operated within a broad and equivalent range of structures, products, processes, and uses without effecting the scope of the invention or any embodiment thereof.

## Claims

**Claims for the following Contracting States : BE, CH, DE, FR, GB, GR, IT, LI, LU, NL, SE**

1. A recombinant DNA (rDNA) molecule comprising a DNA sequence coding for genomic glutamine synthetase as shown in figure 4.

2. A recombinant DNA (rDNA) molecule comprising a DNA sequence of introns and exons coding for genomic glutamine synthetase, wherein said introns indicated by $I^1$- $I^{13}$ and said exons coding for glutamine synthatase comprise the following polypeptide formula:

$I^{1}$-Met-Ser-Leu-Leu-Ser-Asp-Leu-Ile-Asn-
Leu-Asp-Leu-Ser-Glu-Thr-Thr-Glu-Lys-
Ile-Ile-Ala-Glu-Tyr-Ile-Tr-$I^{2}$-p-Ile-Gly-
Gly-Ser-Gly-Leu-Asp-Leu-Arg-Ser-Lys-
Ala-Arg-$I^{3}$-Thr-Leu-Pro-Gly-Pro-Val-Thr-
Asp-Pro-Ser-Gln-Leu-Pro-Lys-Trp-Asn-
Tyr-Asp-Gly-Ser-Ser-Thr-Gly-Gln-Ala-
Pro-Gly-Glu-Asp-Ser-Glu-Val-Ile-Ile-
Ty-$I^{4}$-r-Pro-Gln-Ala-Ile-Phe-Lys-Asp-Pro-
Phe-Arg-Arg-Gly-Asn-Asn-Ile-Leu-$I^{5}$-Val-
Met-Cys-Asp-Ala-Tyr-Thr-Pro-Ala-Gly-
Glu-Pro-Ile-Pro-Thr-Asn-Lys-Arg-His-
Ala-Ala-Ala-Lys-Ile-Phe-Ser-His-Pro-
Asp-Val-Val-Ala-Glu-Val-Pro-Tr-$I^{6}$-p-Tyr-
Gly-Ile-Glu-Gln-Glu-Tyr-Thr-Leu-Leu-
Gln-Lys-Asp-Ile-Asn-Trp-Pro-Leu-Gly-
Trp-Pro-Val-Gly-Gly-Phe-Pro-Gly-Pro-
Gln-$I^{7}$-Gly-Pro-Tyr-Tyr-Cys-Gly-Ala-Gly-
Ala-Asp-Lys-Ala-Phe-Gly-Arg-Asp-Ile-
Val-Asp-Ser-His-Tyr-Lys-Ala-Cys-Leu-
Tyr-Ala-Gly-Ile-Asn-Ile-Ser-Gly-Ile-
Asn-Gly-Glu-Val-Met-Pro-Gly-Gln-$I^{8}$-Trp-
Glu-Phe-Gln-Val-Gly-Pro-Ser-Val-Gly-
Ile-Ser-Ala-Gly-Asp-Glu-Ile-Trp-Val-
Ala-Arg-Tyr-Ile-Leu-Glu-$I^{9}$-Arg-Ile-Thr-
Glu-Val-Ala-Gly-Val-Val-Leu-Ser-Phe-
Asp-Pro-Lys-Pro-Ile-Lys-$I^{10}$-Gly-Asp-Trp-
Asn-Gly-Ala-Gly-Ala-His-Thr-Asn-Tyr-
Se-$I^{11}$-r-Thr-Lys-Ser-Met-Arg-Glu-Asp-Gly-
Gly-Tyr-Glu-Val-Ile-Leu-Lys-Ala-Ile-
Glu-Lys-Leu-Gly-Lys-Lys-His-Lys-Glu-
His-Ile-Ala-Ala-Tyr-Gly-Glu-Gly-Asn-
Glu-Arg-Arg-Leu-Thr-Gly-Arg-His-Glu-

Thr-Ala-Asp-Ile-Asn-Thr-Phe-Leu-Trp-$I^{12}$-
Gly-Val-Ala-Asn-Arg-Gly-Ala-Ser-Ile-
Arg-Val-Gly-Arg-Asp-Thr-Glu-Lys-Ala-
Gly-Lys-Gly-Tyr-Phe-Glu-Asp-Arg-Arg-
Pro-Ser-Ser-Asn-Met-Asp-Pro-Tyr-Val-
Val-Thr-Ser-Met-Ile-Ala-Asp-Thr-Thr-
Ile-Leu-Trp-Lys-Pro-$I^{13}$

wherein $I^1$ is a first intron located before a first exon, $I^{13}$ ia a last intron located after a last exon, $I^2$-$I^{12}$ are introns located between the remaining exons, and wherein each of said introns comprise about ten to 750 nucleotides.

**Claim for the following Contracting States : AT, ES**

**1.** A method for preparing a DNA sequence combination operable in a given plant cell which comprises combining
(a) a first DNA sequence coding for genomic glutamine synthetase (GS) functional in said plant cell, operably linked to
(b) a second DNA sequence capable of increasing the levels of expression of said first DNA sequence such that when such combination is present in an otherwise herbicidal GS-inhibitor sensitive plant cell, said cell is substantially resistant to said herbicidal GS inhibitor
and wherein the first DNA sequence codes for the following polypeptide formula:.

I$^{1}$-Met-Ser-Leu-Leu-Ser-Asp-Leu-Ile-Asn-
Leu-Asp-Leu-Ser-Glu-Thr-Thr-Glu-Lys-
Ile-Ile-Ala-Glu-Tyr-Ile-Tr-I$^{2}$-p-Ile-Gly-
Gly-Ser-Gly-Leu-Asp-Leu-Arg-Ser-Lys-
Ala-Arg-I$^{3}$-Thr-Leu-Pro-Gly-Pro-Val-Thr-
Asp-Pro-Ser-Gln-Leu-Pro-Lys-Trp-Asn-
Tyr-Asp-Gly-Ser-Ser-Thr-Gly-Gln-Ala-
Pro-Gly-Glu-Asp-Ser-Glu-Val-Ile-Ile-
Ty-I$^{4}$-r-Pro-Gln-Ala-Ile-Phe-Lys-Asp-Pro-
Phe-Arg-Arg-Gly-Asn-Asn-Ile-Leu-I$^{5}$-Val-
Met-Cys-Asp-Ala-Tyr-Thr-Pro-Ala-Gly-
Glu-Pro-Ile-Pro-Thr-Asn-Lys-Arg-His-
Ala-Ala-Ala-Lys-Ile-Phe-Ser-His-Pro-
Asp-Val-Val-Ala-Glu-Val-Pro-Tr-I$^{6}$-p-Tyr-
Gly-Ile-Glu-Gln-Glu-Tyr-Thr-Leu-Leu-
Gln-Lys-Asp-Ile-Asn-Trp-Pro-Leu-Gly-
Trp-Pro-Val-Gly-Gly-Phe-Pro-Gly-Pro-
Gln-I$^{7}$-Gly-Pro-Tyr-Tyr-Cys-Gly-Ala-Gly-
Ala-Asp-Lys-Ala-Phe-Gly-Arg-Asp-Ile-
Val-Asp-Ser-His-Tyr-Lys-Ala-Cys-Leu-
Tyr-Ala-Gly-Ile-Asn-Ile-Ser-Gly-Ile-
Asn-Gly-Glu-Val-Met-Pro-Gly-Gln-I$^{8}$-Trp-
Glu-Phe-Gln-Val-Gly-Pro-Ser-Val-Gly-
Ile-Ser-Ala-Gly-Asp-Glu-Ile-Trp-Val-
Ala-Arg-Tyr-Ile-Leu-Glu-I$^{9}$-Arg-Ile-Thr-
Glu-Val-Ala-Gly-Val-Val-Leu-Ser-Phe-
Asp-Pro-Lys-Pro-Ile-Lys-I$^{10}$-Gly-Asp-Trp-
Asn-Gly-Ala-Gly-Ala-His-Thr-Asn-Tyr-
Se-I$^{11}$-r-Thr-Lys-Ser-Met-Arg-Glu-Asp-Gly-Gly-Tyr-Glu-Val-Ile-Leu-Lys-Ala-Ile-
Glu-Lys-Leu-Gly-Lys-Lys-His-Lys-Glu-
His-Ile-Ala-Ala-Tyr-Gly-Glu-Gly-Asn-
Glu-Arg-Arg-Leu-Thr-Gly-Arg-His-Glu-

Thr-Ala-Asp-Ile-Asn-Thr-Phe-Leu-Trp-$I^{12}$-
Gly-Val-Ala-Asn-Arg-Gly-Ala-Ser-Ile-
Arg-Val-Gly-Arg-Asp-Thr-Glu-Lys-Ala-
Gly-Lys-Gly-Tyr-Phe-Glu-Asp-Arg-Arg-
Pro-Ser-Ser-Asn-Met-Asp-Pro-Tyr-Val-
Val-Thr-Ser-Met-Ile-Ala-Asp-Thr-Thr-
Ile-Leu-Trp-Lys-Pro-$I^{13}$

wherein $I^1$ is a first intron located before a first exon, $I^{13}$ ia a last intron located after a last exon, $I^2$-$I^{12}$ are introns located between the remaining exons, and wherein each of said introns comprise about ten to 750 nucleotides.

**Patentansprüche**

**Patentansprüche für folgende Vertragsstaaten : BE, CH, DE, FR, GB, GR, IT, LI, LU, NL, SE**

1. Recombinantes DNA (rDNA) Molekül enthaltend eine DNA-Sequenz, die für genomische Glutamin Synthetase, wie in Fig. 4 gezeigt, codiert.

2. Recombinantes DNA (rDNA) Molekül enthaltend eine DNA-Sequenz von Introns und Exons , die für genomische Glutamin Synthetase codieren, wobei die genannten mit $I^1$ - $I^{13}$ bezeichneten Introns und die genannten Exons , die für Glutamin Synthetase codieren, die folgende Peptid-Formel aufweisen:

I[1]-Met-Ser-Leu-Leu-Ser-Asp-Leu-Ile-Asn-
Leu-Asp-Leu-Ser-Glu-Thr-Thr-Glu-Lys-
Ile-Ile-Ala-Glu-Tyr-Ile-Tr-I[2]-p-Ile-Gly-
Gly-Ser-Gly-Leu-Asp-Leu-Arg-Ser-Lys-
Ala-Arg-I[3]-Thr-Leu-Pro-Gly-Pro-Val-Thr-
Asp-Pro-Ser-Gln-Leu-Pro-Lys-Trp-Asn-
Tyr-Asp-Gly-Ser-Ser-Thr-Gly-Gln-Ala-
Pro-Gly-Glu-Asp-Ser-Glu-Val-Ile-Ile-
Ty-I[4]-r-Pro-Gln-Ala-Ile-Phe-Lys-Asp-Pro-
Phe-Arg-Arg-Gly-Asn-Asn-Ile-Leu-I[5]-Val-
Met-Cys-Asp-Ala-Tyr-Thr-Pro-Ala-Gly-
Glu-Pro-Ile-Pro-Thr-Asn-Lys-Arg-His-
Ala-Ala-Ala-Lys-Ile-Phe-Ser-His-Pro-
Asp-Val-Val-Ala-Glu-Val-Pro-Tr-I[6]-p-Tyr-
Gly-Ile-Glu-Gln-Glu-Tyr-Thr-Leu-Leu-
Gln-Lys-Asp-Ile-Asn-Trp-Pro-Leu-Gly-
Trp-Pro-Val-Gly-Gly-Phe-Pro-Gly-Pro-
Gln-I[7]-Gly-Pro-Tyr-Tyr-Cys-Gly-Ala-Gly-
Ala-Asp-Lys-Ala-Phe-Gly-Arg-Asp-Ile-
Val-Asp-Ser-His-Tyr-Lys-Ala-Cys-Leu-
Tyr-Ala-Gly-Ile-Asn-Ile-Ser-Gly-Ile-
Asn-Gly-Glu-Val-Met-Pro-Gly-Gln-I[8]-Trp-
Glu-Phe-Gln-Val-Gly-Pro-Ser-Val-Gly-
Ile-Ser-Ala-Gly-Asp-Glu-Ile-Trp-Val-

Ala-Arg-Tyr-Ile-Leu-Glu-$I^9$-Arg-Ile-Thr-
Glu-Val-Ala-Gly-Val-Val-Leu-Ser-Phe-
Asp-Pro-Lys-Pro-Ile-Lys-$I^{10}$-Gly-Asp-Trp-
Asn-Gly-Ala-Gly-Ala-His-Thr-Asn-Tyr-
Se-$I^{11}$-r-Thr-Lys-Ser-Met-Arg-Glu-Asp-Gly-
Gly-Tyr-Glu-Val-Ile-Leu-Lys-Ala-Ile-
Glu-Lys-Leu-Gly-Lys-Lys-His-Lys-Glu-
His-Ile-Ala-Ala-Tyr-Gly-Glu-Gly-Asn-
Glu-Arg-Arg-Leu-Thr-Gly-Arg-His-Glu-
Thr-Ala-Asp-Ile-Asn-Thr-Phe-Leu-Trp-$I^{12}$-
Gly-Val-Ala-Asn-Arg-Gly-Ala-Ser-Ile-
Arg-Val-Gly-Arg-Asp-Thr-Glu-Lys-Ala-
Gly-Lys-Gly-Tyr-Phe-Glu-Asp-Arg-Arg-
Pro-Ser-Ser-Asn-Met-Asp-Pro-Tyr-Val-
Val-Thr-Ser-Met-Ile-Ala-Asp-Thr-Thr-
Ile-Leu-Trp-Lys-Pro-$I^{13}$

in welcher $I^1$ ein erstes Intron ist, das vor einem ersten Exon angeordnet ist, $I^{13}$ ein letztes Intron ist, das nach einem letzten Exon angeordnet ist, $I^2$ - $I^{12}$ Introns sind, die zwischen den verbleibenden Exons angeordnet sind, und wobei jedes der genannten Introns etwa 10 bis 750 Nucleotide umfaßt.

**Patentanspruch für folgende Vertragsstaaten : AT, ES**

**1.** Verfahren zur Herstellung einer DNA-Sequenz-Kombination, die in einer vorgegebenen Pflanzenzelle operabel ist, umfassend das Kombinieren von

(a) einer ersten DNA-Sequenz, die für genomische Glutamin Synthetase (GS) codiert, die in der genannten Pflanzenzelle funktional ist, operabel gekoppelt an

(b) eine zweite DNA-Sequenz, die geeignet ist die Expressionswerte der genannten ersten DNA-Sequenz zu steigern, so daß, wenn eine solche Kombination in einer ansonsten gegenüber einem herbiziden GS-Inhibitor sensitiven Pflanzenzelle vorhanden ist, die genannte Zelle im wesentlichen gegenüber dem genannten herbiziden GS-Inhibitor resistent ist, und wobei die erste DNA-Sequenz für die folgende Peptidformel codiert:

$I^1$-Met-Ser-Leu-Leu-Ser-Asp-Leu-Ile-Asn-
Leu-Asp-Leu-Ser-Glu-Thr-Thr-Glu-Lys-
Ile-Ile-Ala-Glu-Tyr-Ile-Tr-$I^2$-p-Ile-Gly-
Gly-Ser-Gly-Leu-Asp-Leu-Arg-Ser-Lys-
Ala-Arg-$I^3$-Thr-Leu-Pro-Gly-Pro-Val-Thr-
Asp-Pro-Ser-Gln-Leu-Pro-Lys-Trp-Asn-
Tyr-Asp-Gly-Ser-Ser-Thr-Gly-Gln-Ala-
Pro-Gly-Glu-Asp-Ser-Glu-Val-Ile-Ile-
Ty-$I^4$-r-Pro-Gln-Ala-Ile-Phe-Lys-Asp-Pro-
Phe-Arg-Arg-Gly-Asn-Asn-Ile-Leu-$I^5$-Val-
Met-Cys-Asp-Ala-Tyr-Thr-Pro-Ala-Gly-
Glu-Pro-Ile-Pro-Thr-Asn-Lys-Arg-His-
Ala-Ala-Ala-Lys-Ile-Phe-Ser-His-Pro-
Asp-Val-Val-Ala-Glu-Val-Pro-Tr-$I^6$-p-Tyr-
Gly-Ile-Glu-Gln-Glu-Tyr-Thr-Leu-Leu-
Gln-Lys-Asp-Ile-Asn-Trp-Pro-Leu-Gly-
Trp-Pro-Val-Gly-Gly-Phe-Pro-Gly-Pro-
Gln-$I^7$-Gly-Pro-Tyr-Tyr-Cys-Gly-Ala-Gly-
Ala-Asp-Lys-Ala-Phe-Gly-Arg-Asp-Ile-
Val-Asp-Ser-His-Tyr-Lys-Ala-Cys-Leu-
Tyr-Ala-Gly-Ile-Asn-Ile-Ser-Gly-Ile-

Asn-Gly-Glu-Val-Met-Pro-Gly-Gln-$I^{8}$-Trp-
Glu-Phe-Gln-Val-Gly-Pro-Ser-Val-Gly-
Ile-Ser-Ala-Gly-Asp-Glu-Ile-Trp-Val-
Ala-Arg-Tyr-Ile-Leu-Glu-$I^{9}$-Arg-Ile-Thr-
Glu-Val-Ala-Gly-Val-Val-Leu-Ser-Phe-
Asp-Pro-Lys-Pro-Ile-Lys-$I^{10}$-Gly-Asp-Trp-
Asn-Gly-Ala-Gly-Ala-His-Thr-Asn-Tyr-
Se-$I^{11}$-r-Thr-Lys-Ser-Met-Arg-Glu-Asp-Gly-
Gly-Tyr-Glu-Val-Ile-Leu-Lys-Ala-Ile-
Glu-Lys-Leu-Gly-Lys-Lys-His-Lys-Glu-
His-Ile-Ala-Ala-Tyr-Gly-Glu-Gly-Asn-
Glu-Arg-Arg-Leu-Thr-Gly-Arg-His-Glu-

Thr-Ala-Asp-Ile-Asn-Thr-Phe-Leu-Trp-$I^{12}$-
Gly-Val-Ala-Asn-Arg-Gly-Ala-Ser-Ile-
Arg-Val-Gly-Arg-Asp-Thr-Glu-Lys-Ala-
Gly-Lys-Gly-Tyr-Phe-Glu-Asp-Arg-Arg-
Pro-Ser-Ser-Asn-Met-Asp-Pro-Tyr-Val-
Val-Thr-Ser-Met-Ile-Ala-Asp-Thr-Thr-
Ile-Leu-Trp-Lys-Pro-$I^{13}$

in welcher $I^1$ ein erstes Intron ist, das vor einem ersten Exon angeordnet ist, $I^{13}$ ein letztes Intron ist, das nach einem letzten Exon angeordnet ist, $I^2$ - $I^{12}$ Introns sind, die zwischen den verbleibenden Exons angeordnet sind, und wobei jedes der genannten Introne etwa 10 bis 750 Nucleotide umfaßt.

## Revendications

**Revendications pour les Etats contractants suivants : BE, CH, DE, FR, GB, GR, IT, LI, LU, NL, SE**

1. Molécule d'ADN recombinant (ADNr) comprenant une séquence d'ADN codant pour la glutamine synthétase génomique telle que présenté dans la figure 4.

2. Molécule d'ADN recombinant (ADNr) comprenant une séquence d'ADN d'introns et d'exons codant pour la glutamine synthétase génomique, dans laquelle lesdits introns indiqués par $I^1$ à $I^{13}$ et lesdits exons codant pour la glutamine synthétase comprennent la formule polypeptidique suivante :

$I^{1}$-Met-Ser-Leu-Leu-Ser-Asp-Leu-Ile-Asn-
Leu-Asp-Leu-Ser-Glu-Thr-Thr-Glu-Lys-
Ile-Ile-Ala-Glu-Tyr-Ile-Tr-$I^{2}$-p-Ile-Gly-
Gly-Ser-Gly-Leu-Asp-Leu-Arg-Ser-Lys-
Ala-Arg-$I^{3}$-Thr-Leu-Pro-Gly-Pro-Val-Thr-
Asp-Pro-Ser-Gln-Leu-Pro-Lys-Trp-Asn-
Tyr-Asp-Gly-Ser-Ser-Thr-Gly-Gln-Ala-
Pro-Gly-Glu-Asp-Ser-Glu-Val-Ile-Ile-
Ty-$I^{4}$-r-Pro-Gln-Ala-Ile-Phe-Lys-Asp-Pro-
Phe-Arg-Arg-Gly-Asn-Asn-Ile-Leu-$I^{5}$-Val-
Met-Cys-Asp-Ala-Tyr-Thr-Pro-Ala-Gly-
Glu-Pro-Ile-Pro-Thr-Asn-Lys-Arg-His
Ala-Ala-Ala-Lys-Ile-Phe-Ser-His-Pro-
Asp-Val-Val-Ala-Glu-Val-Pro-Tr-$I^{6}$-p-Tyr-
Gly-Ile-Glu-Gln-Glu-Tyr-Thr-Leu-Leu-
Gln-Lys-Asp-Ile-Asn-Trp-Pro-Leu-Gly-
Trp-Pro-Val-Gly-Gly-Phe-Pro-Gly-Pro-
Gln-$I^{7}$-Gly-Pro-Tyr-Tyr-Cys-Gly-Ala-Gly-
Ala-Asp-Lys-Ala-Phe-Gly-Arg-Asp-Ile
Val-Asp-Ser-His-Tyr-Lys-Ala-Cys-Leu-
Tyr-Ala-Gly-Ile-Asn-Ile-Ser-Gly-Ile
Asn-Gly-Glu-Val-Met-Pro-Gly-Gln-$I^{8}$-Trp-

Glu-Phe-Gln-Val-Gly-Pro-Ser-Val-Gly-
Ile-Ser-Ala-Gly-Asp-Glu-Ile-Trp-Val-
Ala-Arg-Tyr-Ile-Leu-Glu-$I^{9}$-Arg-Ile-Thr-
Glu-Val-Ala-Gly-Val-Val-Leu-Ser-Phe-
Asp-Pro-Lys-Pro-Ile-Lys-$I^{10}$-Gly-Asp-Trp-
Asn-Gly-Ala-Gly-Ala-His-Thr-Asn-Tyr-
Se-$I^{11}$-r-Thr-Lys-Ser-Met-Arg-Glu-Asp-Gly-
Gly-Tyr-Glu-Val-Ile-Leu-Lys-Ala-Ile-
Glu-Lys-Leu-Gly-Lys-Lys-His-Lys-Glu-
His-Ile-Ala-Ala-Tyr-Gly-Glu-Gly-Asn-
Glu-Arg-Arg-Leu-Thr-Gly-Arg-His-Glu-
Thr-Ala-Asp-Ile-Asn-Thr-Phe-Leu-Trp-$I^{12}$-
Gly-Val-Ala-Asn-Arg-Gly-Ala-Ser-Ile-
Arg-Val-Gly-Arq-Asp-Thr-Glu-Lys-Ala-
Gly-Lys-Gly-Thr-Phe-Glu-Asp-Arg-Arg-
Pro-Ser-Ser-Asn-Met-Asp-Pro-Tyr-Val-
Val-Thr-Ser-Met-Ile-Ala-Asp-Thr-Thr-
Ile-Leu-Trp-Lys-Pro-$I^{13}$

dans laquelle $I^1$ est un premier intron situé avant un premier exon, $I^{13}$ est un dernier intron situé après un dernier exon, $I^2$ à $I^{12}$ sont des introns situés entre les exons restant, et dans laquelle chacun desdits introns comprend d'environ 10 à 750 nucléotides.

**Revendication pour les Etats contractants suivants : AT, ES**

1. Procédé pour préparer une combinaison de séquences utilisable dans une cellule végétale donnée qui comprend la combinaison
   (a) d'une première séquence d'ADN codant pour la glutamine synthétase génomique (GS) fonctionnelle dans ladite cellule végétale, liée de façon utilisable à
   (b) une seconde séquence d'ADN capable d'augmenter les niveaux d'expression de ladite première séquence d'ADN de façon que quand une telle combinaison est présente dans une cellule végétale par ailleurs sensible à un inhibiteur herbicide de la GS, ladite cellule est sensiblement résistante audit inhibiteur herbicide de la GS

   et dans lequel la première séquence d'ADN code pour la formule polypeptidique suivante :

$I^1$-Met-Ser-Leu-Leu-Ser-Asp-Leu-Ile-Asn-
Leu-Asp-Leu-Ser-Glu-Thr-Thr-Glu-Lys-
Ile-Ile-Ala-Glu-Tyr-Ile-Tr-$I^2$-p-Ile-Gly-
Gly-Ser-Gly-Leu-Asp-Leu-Arg-Ser-Lys-
Ala-Arg-$I^3$-Thr-Leu-Pro-Gly-Pro-Val-Thr-
Asp-Pro-Ser-Gln-Leu-Pro-Lys-Trp-Asn-
Tyr-Asp-Gly-Ser-Ser-Thr-Gly-Gln-Ala-
Pro-Gly-Glu-Asp-Ser-Glu-Val-Ile-Ile-
Ty-$I^4$-r-Pro-Gln-Ala-Ile-Phe-Lys-Asp-Pro-
Phe-Arg-Arg-Gly-Asn-Asn-Ile-Leu-$I^5$-Val-
Met-Cys-Asp-Ala-Tyr-Thr-Pro-Ala-Gly-
Glu-Pro-Ile-Pro-Thr-Asn-Lys-Arg-His
Ala-Ala-Ala-Lys-Ile-Phe-Ser-His-Pro-
Asp-Val-Val-Ala-Glu-Val-Pro-Tr-$I^6$-p-Tyr-
Gly-Ile-Glu-Gln-Glu-Tyr-Thr-Leu-Leu-
Gln-Lys-Asp-Ile-Asn-Trp-Pro-Leu-Gly-
Trp-Pro-Val-Gly-Gly-Phe-Pro-Gly-Pro-

Gln-$I^7$-Gly-Pro-Tyr-Tyr-Cys-Gly-Ala-Gly-
Ala-Asp-Lys-Ala-Phe-Gly-Arg-Asp-Ile
Val-Asp-Ser-His-Tyr-Lys-Ala-Cys-Leu-
Tyr-Ala-Gly-Ile-Asn-Ile-Ser-Gly-Ile
Asn-Gly-Glu-Val-Met-Pro-Gly-Gln-$I^8$-Trp-
Glu-Phe-Gln-Val-Gly-Pro-Ser-Val-Gly-
Ile-Ser-Ala-Gly-Asp-Glu-Ile-Trp-Val-
Ala-Arg-Tyr-Ile-Leu-Glu-$I^9$-Arg-Ile-Thr-
Glu-Val-Ala-Gly-Val-Val-Leu-Ser-Phe-
Asp-Pro-Lys-Pro-Ile-Lys-$I^{10}$-Gly-Asp-Trp-
Asn-Gly-Ala-Gly-Ala-His-Thr-Asn-Tyr-
Se-$I^{11}$-r-Thr-Lys-Ser-Met-Arg-Glu-Asp-Gly-
Gly-Tyr-Glu-Val-Ile-Leu-Lys-Ala-Ile-
Glu-Lys-Leu-Gly-Lys-Lys-His-Lys-Glu-
His-Ile-Ala-Ala-Tyr-Gly-Glu-Gly-Asn-
Glu-Arg-Arg-Leu-Thr-Gly-Arg-His-Glu-
Thr-Ala-Asp-Ile-Asn-Thr-Phe-Leu-Trp-$I^{12}$-
Gly-Val-Ala-Asn-Arg-Gly-Ala-Ser-Ile-
Arg-Val-Gly-Arq-Asp-Thr-Glu-Lys-Ala-
Gly-Lys-Gly-Thr-Phe-Glu-Asp-Arg-Arg-
Pro-Ser-Ser-Asn-Met-Asp-Pro-Tyr-Val-
Val-Thr-Ser-Met-Ile-Ala-Asp-Thr-Thr-
Ile-Leu-Trp-Lys-Pro-$I^{13}$

dans laquelle $I^1$ est un premier intron situé avant un premier exon, $I^{13}$ est un dernier intron situé après un dernier exon, $I^2$ à $I^{12}$ sont des introns situés entre les exons restants, et dans laquelle chacun dedits introns comprend d'environ 10 à 750 nucléotides.

*Fig. 1*

*THE GLUTAMATE SYNTHASE CYCLE*

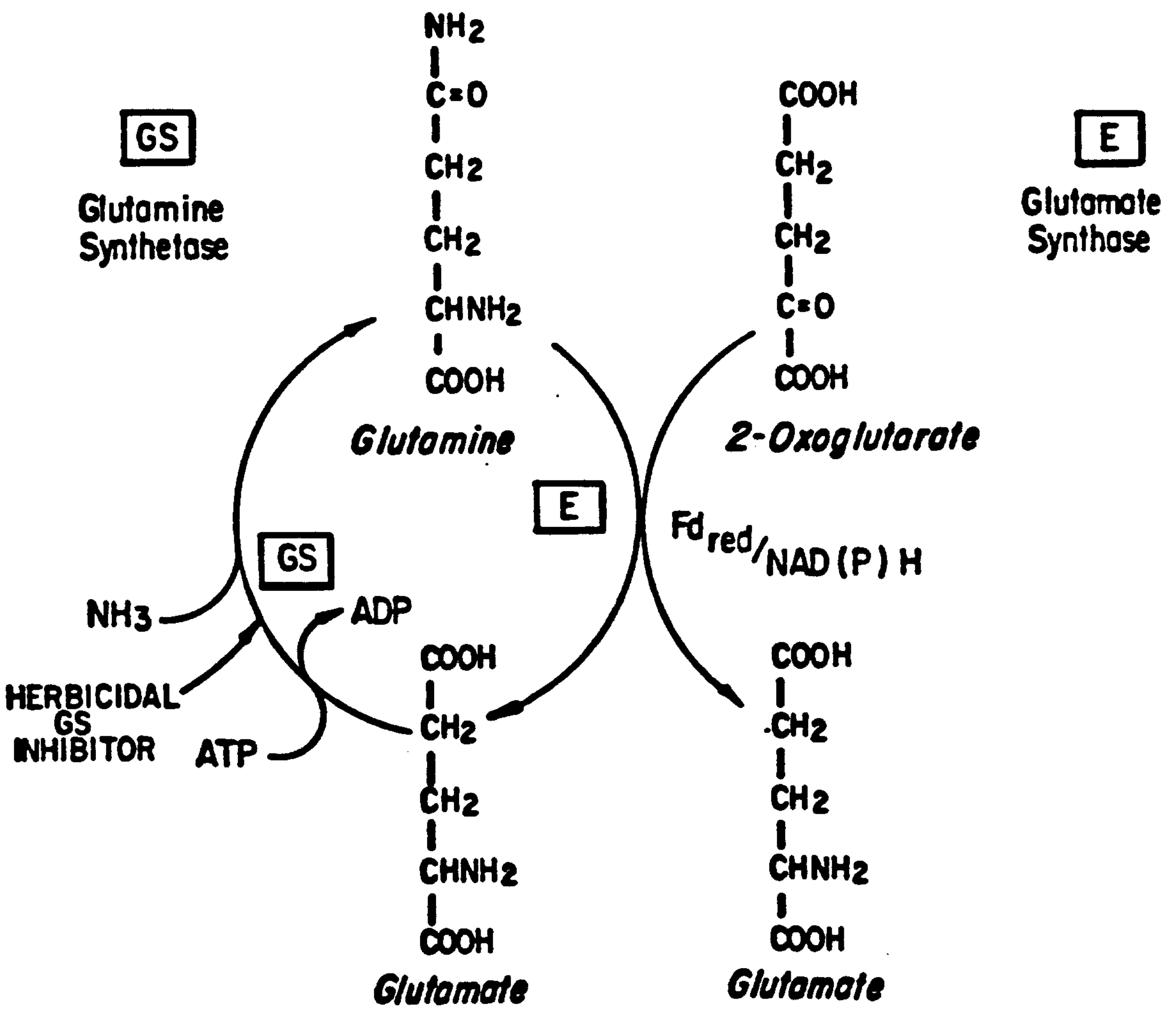

*Fig.2*

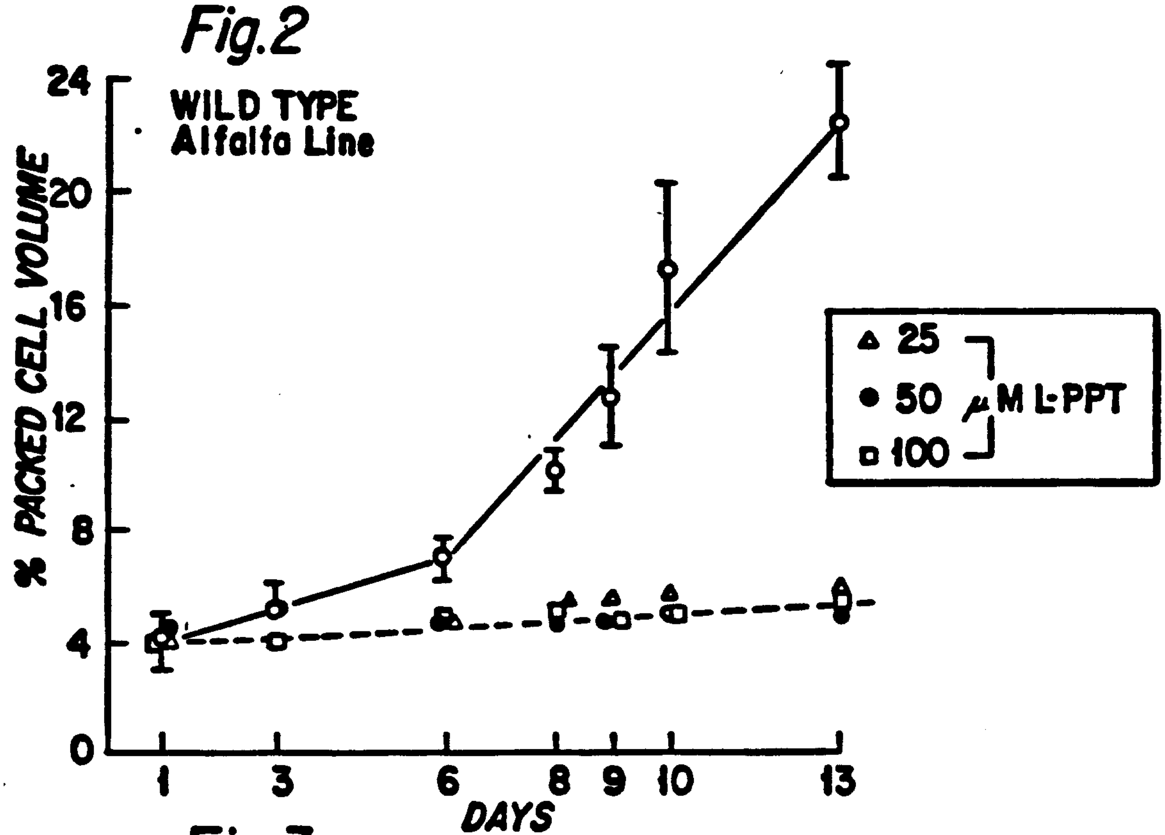
WILD TYPE
Alfalfa Line
% PACKED CELL VOLUME
24
20
16
12
8
4
0
1
3
6
8
9
10
13
DAYS
▵ 25
● 50 μM L-PPT
□ 100

*Fig.3*

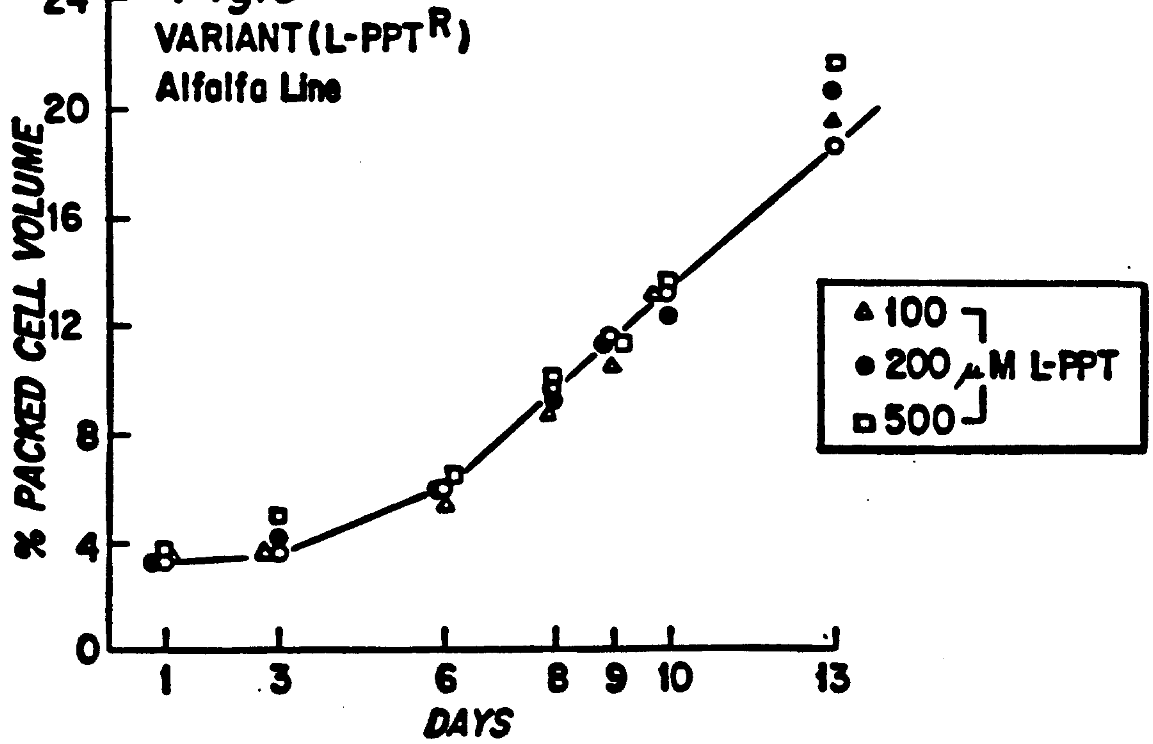
VARIANT (L-PPT^R)
Alfalfa Line
% PACKED CELL VOLUME
24
20
16
12
8
4
0
1
3
6
8
9
10
13
DAYS
▵ 100
● 200 μM L-PPT
□ 500

Fig. 4